# EUROPEAN PATENT APPLICATION

(11) **EP 2 186 481 A1**
(43) Date of publication of application: **19.05.2010**
(21) Application number: 09176071.0
(22) Date of filing: 16.11.2009
(51) Int. Cl.: A61B 8/00, A61B 8/08, G10K 11/35, G01S 15/89

(54) **Ultrasonic probe capable of probing curved surface**

(30) Priority: 17.11.2008 KR 20080113915
(71) Applicant: MEDISON CO., LTD., Kangwon-do 250-870 (KR)
(72) Inventor: Kim, Seong Rae, 135-851, Seoul (KR); Kim, Jae Kyung, 135-851, Seoul (KR); Kim, Jong Sik, 135-851, Seoul (KR)
(74) Representative: Schmid, Wolfgang

(57) **Abstract**

An ultrasonic probe capable of probing a curved surface is disclosed in one embodiment in accordance with the disclosure as including a housing, a transducer, a guide part and a drive part. The transducer is disposed in the housing. The transducer is movable alongside the curved surface of the examined part. The guide part is configured to guide the transducer along the curved surface of the examined part. The drive part is configured to move the transducer along the curved surface of the examined part. According to the disclosure, the ultrasonic probe can probe or scan a curved examined part of a subject such as a neck, a breast, etc.

## Description

The present application claims priority to Korean Patent Application No. 10-2008-0113915 filed on November 17, 2008, the entire disclosure of which is incorporated herein by reference.

### TECHNICAL FIELD

The present disclosure relates to an ultrasonic probe adapted to probe or scan a curved examined part of a subject's body such as a neck, a breast, etc.

### BACKGROUND

An ultrasonic diagnostic apparatus has been widely used as a medical imaging instrument for displaying and diagnosing internal images of a subject's body. Generally, the ultrasonic diagnostic apparatus performs an ultrasonic diagnosis by using an ultrasonic probe configured to radiate ultrasonic waves into an examined part of a subjects' body and receive echo signals therefrom. The ultrasonic probe includes a transducer with a number of ultrasonic oscillators.

A conventional ultrasonic probe includes a flat probing surface at its fore end. The transducer is positioned below the flat probing surface. When using the ultrasonic probe with the flat probing surface to probe or scan a curved examined part of a subject's body (e.g., a neck), it is difficult to probe such a part along its curved surface as the probing surface becomes closely contacted to the curved examined part. Further, although the ultrasonic images for the curved examined part can be acquired by the conventional ultrasonic probe with the flat probing surface, such images might be discontinuous or non-uniform. Thus, a precise medical diagnosis can be hardly performed by such discontinuous or non-uniform ultrasonic images. Further, a subject must lie on a bed, for example, so that an operator or sonographer of the ultrasonic diagnostic apparatus can bring the ultrasonic probe into close contact with the curved examined part when probing the curved examined part. Thus, the subject may feel inconvenient and uncomfortable.

Especially, in the context of telemedicine recently introduced in the art, it is possible that a sonographer of the ultrasonic diagnostic apparatus does not exactly comprehend what a remote medical team wants. Thus, the discontinuous or non-uniform images, which can be acquired by the conventional ultrasonic probe with the flat probing surface, cannot provide sufficient information to achieve telemedicine.

### SUMMARY

Various embodiments of an ultrasonic probe for use with an ultrasonic diagnostic apparatus are provided. In one embodiment of the present disclosure, by way of nonlimiting example, an ultrasonic probe comprises: a housing; a transducer disposed in the housing so as to move alongside a curved surface of an examined part; and a guide part configured to guide the transducer along the curved surface of the examined part.

The housing may define an opening opened toward the examined part. An ultrasonic medium, which is deformable in conformity with the curved surface of the examined part, may be positioned between the transducer and the examined part via the opening. Thus, one side of the ultrasonic medium may be in contact with the transducer, while an opposite side of the ultrasonic medium may be in contact with the curved surface of the examined part.

The ultrasonic medium may comprise an elastic bag filled with an ultrasonic conductivity couplant. Alternatively, the ultrasonic medium may comprise an elastic solid object of a rubber-like ultrasonic conductivity material such as silicone rubber.

The guide part may include a first guide element provided at the housing and a second guide element provided at the transducer. The first and second guide elements may be engaged to each other in order to guide the transducer.

The ultrasonic probe further comprises a drive part configured to move the transducer along the curved surface of the examined part. The drive part may include a roller and a circular-arc-shaped roller guide rail on which the roller rolls. Thus, as the drive part rotates the roller, the roller moves along the roller guide rail to move the transducer as guided by the guide part.

Alternatively, the drive part may include a pinion gear and a circular-arc-shaped rack gear meshed with the pinion gear. Thus, as the drive part rotates the pinion gear, the pinion gear moves along the rack gear to move the transducer as guided by the guide part.

The ultrasonic probe may further comprise a jaw rest configured to rest a subject's jaw thereon and a shoulder support to support a subject's shoulder thereon.

The Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

### BRIEF DESCRIPTION OF THE DRAWINGS

Arrangements and embodiments may be described in detail with reference to the following drawings in which like reference numerals refer to like elements and wherein:
- FIG. 1: is a side view illustrating an ultrasonic probe capable of probing a curved surface according to one embodiment of the present disclosure;
- FIG. 2: is a top sectional view illustrating an interior of the ultrasonic probe with an ultrasonic medium being in contact with a neck part of a subject;
- FIG. 3: schematically illustrates the procedure of probing a subject's neck using the ultrasonic probe shown in FIG. 1;
- FIG. 4: is a partially enlarged view of FIG. 2, which schematically illustrates an embodiment of a drive part of the ultrasonic probe shown in FIG. 1; and
- FIG. 5: schematically illustrates another embodiment of the drive part shown in FIG. 1.

### DETAILED DESCRIPTION

A detailed description may be provided with reference to the accompanying drawings. One of ordinary skill in the art may realize that the following description is illustrative only and is not in any way limiting. Other illustrative embodiments may readily suggest themselves to such skilled persons having the benefit of this disclosure.

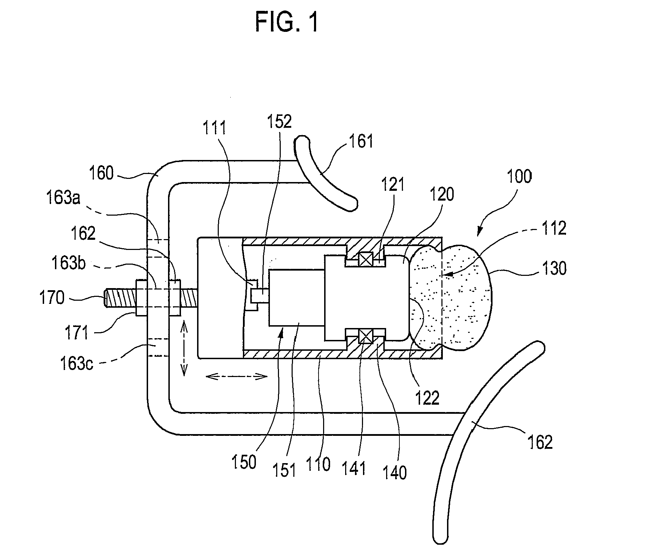

With Reference to FIGS. 1 to 4, an ultrasonic probe capable of probing a curved examined part according to an exemplary embodiment of the present disclosure will be now described. The ultrasonic probe described in FIGS. 1 to 4 may be used to probe or scan around, for example, a neck part in which a thyroid gland exists.

As shown in FIGS. 1 to 3, the ultrasonic probe of this embodiment may be fixed relative to subject's jaw and shoulder to probe or scan around a subjects' neck part with a thyroid gland therein. In this embodiment, the ultrasonic probe 100 capable of probing the neck part may include the following: a housing 110 defining an opening 112 opened toward and concave to the examined part; a transducer 120 disposed in the housing 110 and being configured to be reciprocatingly movable alongside the curved surface of the examined part; an ultrasonic medium 130 mounted to the opening 112 of the housing 110 in contact with a radiation and reception surface 122 of the transducer 120; a drive part 150 configured to reciprocate the transducer 120; and a fixing member 160 configured to contact or engage the housing 110 to a subject having the examined part.

The housing 110 may be configured to surround the curved surface of the examined part of the subject within a predetermined region. The housing 110 may define a circular-arc-shaped internal space therein, wherein the transducer 120 can reciprocate along a circular-arc path. The opening 112 may be defined in the housing 110 such that it is opened toward the examined part and concavely shaped so as to correspond to the curved surface of the examined part.

The transducer 120 may reciprocate along a circular-arc path within the housing 110. The transducer 120 may be guided by a guide part comprising a circular-arc-shaped guide rail 140 and a guide groove 121. The transducer 120 may have a radiation and reception surface 122 at its side or portion facing toward the examined part. Ultrasonic waves may be radiated through the radiation and reception surface 122 and echo signals may be received therethrough. The transducer 120 or the ultrasonic medium 130 may be positioned such that the radiation and reception surface 122 of the transducer 120 can be brought into close contact with one side of the ultrasonic medium 130.

The ultrasonic medium 130 may be in close contact with the examined part so as not to generate noises during ultrasonic diagnosis. The ultrasonic medium 130 may include an elastic bag filled with ultrasonic couplant. In such a case, the ultrasonic couplant may include, but is not limited to, an ultrasonic conductivity gel that can be applied on a surface of the examined part in typical ultrasonic diagnosis. Alternatively, the ultrasonic medium 130 may include an object that can transmit ultrasonic waves and may be conformably deformable to be placed into close contact with the curved surface of the examined part. By way of an example, the ultrasonic medium 130 may include an object that comprises an ultrasound-transmitting rubber-like material such as a silicone rubber and is deformable in conformity with a profile of the curved surface of the examined part.

The ultrasonic medium 130 may be fixed to the opening 112 of the housing 110 and at least partially exposed toward the examined part through the opening 112 of the housing 110. The elasticity of the ultrasonic medium 130 may allow the exposed portion of the ultrasonic medium 130 to conformably be in contact with the curved surface of the examined part. When the opening 112 of the housing 110 is placed on the subject's neck part, the exposed portion of the ultrasonic medium 130 may be in contact with and cover the curved surface of the subject's neck part.

The housing may have the circular-arc-shaped guide rail 140 at the rear of the ultrasonic medium 130. The circular-arc-shaped guide rail 140 may be concave to the examined part so as to correspond to the curved surface of the examined part. A bearing element 141 such as rolling bearings may be disposed on the circular-arc-shaped guide rail 140 along a length direction thereof. The guide groove 121 corresponding to the circular-arc-shaped guide rail 140 may be defined on the transducer 120. Thus, the transducer 120 can move along the circular-arc-shaped guide rail 140 by cooperation of the circular-arc-shaped guide rail 140 and the guide groove 121.

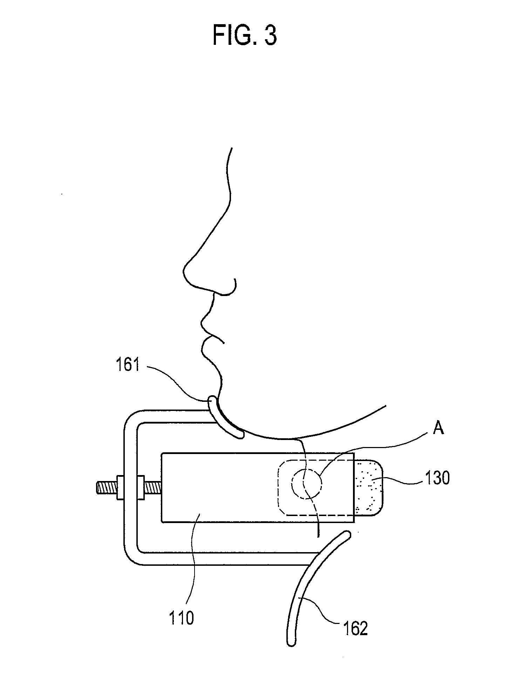

The fixing member 160 may include a frame extending from the housing 110. At both ends of the fixing member 160, a jaw rest 161 and a shoulder support 162 may be provided. As shown in FIG. 3, if the jaw rest 161 may be fixed to a subject's jaw and the shoulder support 162 fixed to a subject's shoulder, then the housing 110 can stay stationary while the ultrasonic medium 130 may be in close contact around the neck part of the subject. The fixing member 160 may be integrally provided to or separated from the housing 110.

Referring back to FIG. 1, by way of an example of coupling arrangement between the fixing member 160 and the housing 110, the fixing member 160 may have a plurality of threaded apertures 163a, 163b, and 163c arranged vertically. The fixing member 160 may be fixed to the housing 110 in such a manner that a screw or bolt 170 is coupled to one of the threaded apertures 163a, 163b, and 163c. Further, a pair of nuts 171 and 172 may be fastened to either side of the bolt 170 to adjust a horizontal position of the bolt 170. Thus, the position of the fixing member 160 can be adjusted vertically or horizontally relative to the housing 110.

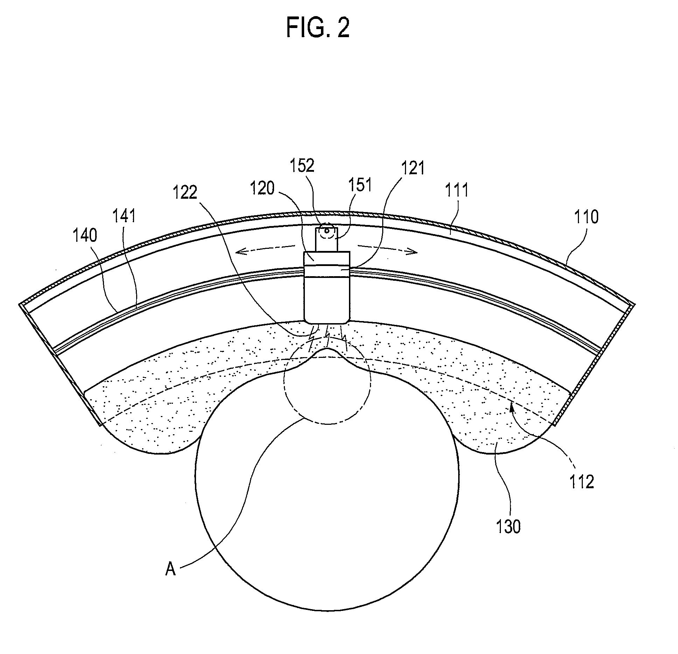

As shown in FIGS. 2 and 3, the ultrasonic medium 130 may be in contact with and cover the curved surface of an examined part A due to its own elasticity. The transducer 120 reciprocates along the circular-arc-shaped guide rail 140 as being in contact with the rear surface of the ultrasonic medium 130 via its radiation and reception surface 122, and thus probes or scans the examined part A. As such, since the transducer 120 reciprocates along the circular-arc-shaped guide rail 140, the examined part A with a curved surface can be easily probed or scanned and continuous ultrasonic images for the inside of the examined part A can be acquired with a uniform quality.

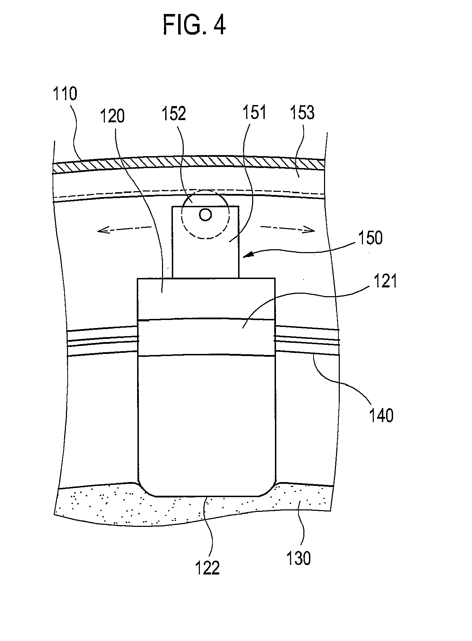

As shown in FIG. 4, in one embodiment, the drive part 150 may include a drive motor 151, which serves as a drive source of the drive part 150, and a roller 152 driven by the drive motor 151 to roll thereby. The drive motor 151 may be coupled to a rear side of the transducer 120. The drive part 150 may include a roller guide rail 153 serving as a part, which the roller 152 is placed into contact with and rolls along. The roller guide rail 153 may be disposed at an inner (or innermost) side of the housing 110. The roller guide rail 153 may have a circular-arc shape concentrical with the circular-arc-shaped guide rail 140. Thus, as the drive motor 151 rolls the roller 152, the roller 152 may travel along the roller guide rail 153 with the help of friction between the roller 152 and the roller guide rail 153, and the transducer 120 may thus be moved along the circular-arc-shaped guide rail 140.

To reduce noises generated during ultrasonic diagnosis, an ultrasonic conductivity gel or oil may be applied on either the ultrasonic medium 130 or the curved surface of the examined part A. Also, such gel or oil may be applied between the radiation and reception surface 122 of the transducer 120 and the ultrasonic medium 130 for purposes of lubrication and noise reduction.

According to the ultrasonic probe of the present disclosure, ultrasonic images with uniform quality can be precisely and quickly acquired irrespective of the skill and experience of sonographers or operators. Further, since the fixing member 160 fixes the housing 110 with respect to a subject's jaw and shoulder, the examined part can be probed or scanned as the housing 110 stays stationary. Accordingly, the ultrasonic probe 100 can be suitably applied to a telemedicine wherein a remote medical team examines a subject via a sonographer or operator.

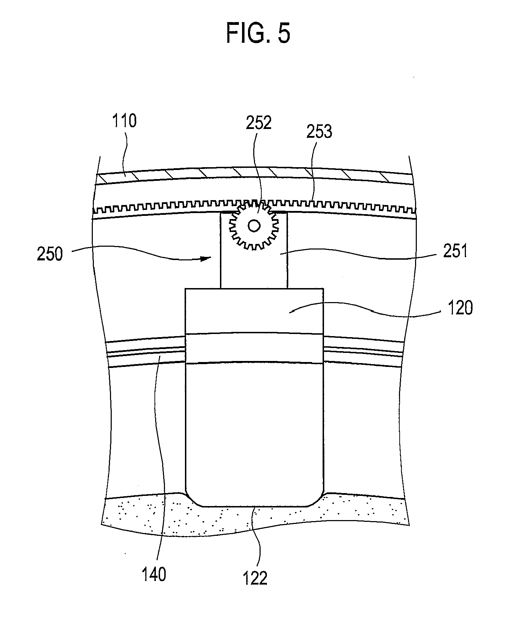

FIG. 5 shows another embodiment of the drive part of the transducer. In this embodiment, the drive part 250 for driving the transducer 120 may include a drive motor 251, a pinion gear 252 configured to be rotated by the drive motor 251, and a rack gear 211 configured to be meshed with the pinion gear 252. The rack gear 211 may be placed in the inner side of the housing 110 and curved concentrically with the circular-arc-shaped guide rail 140, similar to the roller guide rail 153 in the foregoing embodiment. The arrangement of rack and pinion gears may provide a more precise control or movement of the transducer 120 by, for example, varying revolutions of the pinion gear 252.

Although embodiments have been described with reference to a number of illustrative embodiments thereof, it should be understood that various other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasonic probe, comprising:
a housing (110); a transducer (120) disposed in the housing so as to move alongside a curved surface of an examined part; and a guide part (121, 140) configured to guide the transducer along the curved surface of the examined part.

2. The ultrasonic probe of Claim 1, wherein the housing defines an opening (112) configured to be opened toward the examined part and be concave to the curved surface of the examined part, and wherein the ultrasonic probe further comprises an ultrasonic medium (130) disposed at the opening and deformable in conformity with the curved surface of the examined part, the ultrasonic medium being in contact with the transducer at one side thereof.

3. The ultrasonic probe of Claim 2, wherein the ultrasonic medium comprises an elastic bag filled with an ultrasonic conductivity couplant.

4. The ultrasonic probe of Claim 3, wherein the ultrasonic conductivity couplant includes an ultrasonic conductivity gel.

5. The ultrasonic probe of Claim 2, wherein the ultrasonic medium comprises an elastic solid object of a rubber-phase ultrasonic conductivity material.

6. The ultrasonic probe of Claim 5, wherein the ultrasonic conductivity material includes a silicone rubber.

7. The ultrasonic probe of Claim 1, wherein the guide part includes a first guide element (120) provided at the housing and a second guide element (121) provided at the transducer, the first and the second guide elements being engaged to each other to guide the transducer.

8. The ultrasonic probe of Claim 7, wherein the first guide element includes a circular-arc-shaped guide rail (140) extending alongside the curved surface of the examined part and the second guide element includes a circular-arc-shaped guide groove (121) corresponding to the circular-arc-shaped guide rail.

9. The ultrasonic probe of Claim 8, wherein the circular-arc-shaped guide rail has bearings (141) arranged along a length direction thereof.

10. The ultrasonic probe of Claim 1 further comprising a drive part (150) configured to move the transducer along the curved surface of the examined part,
wherein the drive part includes:
a drive motor (151) coupled to the transducer;
a roller (152) configured to be rotated by the drive motor; and
a roller guide rail (153) in rolling contact with the roller and fixed to the housing,
wherein as the drive motor rotates the roller, the roller rolls along the roller guide rail to move the transducer.

11. The ultrasonic probe of Claim 1, further comprising a drive part configured to move the transducer along the curved surface of the examined part,
wherein the drive part includes:
a drive motor (251) coupled to the transducer,
a pinion gear (252) configured to be rotated by the drive motor; and
a rack gear (253) meshed with the pinion gear and fixed to the housing,
wherein as the drive motor rotates the pinion gear, the pinion gear moves along the rack gear to move the transducer.

12. The ultrasonic probe of Claim 1, further comprising a fixing member (160) extending from the housing, the fixing member including a jaw rest (161) configured to rest a subject's jaw thereon and a shoulder support (162) to support a subject's shoulder thereon.
